# EUROPEAN PATENT SPECIFICATION

(11) **EP 1 276 477 B1**
(45) Date of publication and mention of the grant of the patent: **10.12.2003**
(21) Application number: 00958873.2
(22) Date of filing: 12.09.2000
(51) Int. Cl.: A61K 31/195, A61P 19/02

(54) **MEDICAMENTS CONTAINING PANTOTHENIC ACID FOR THE TREATMENT OF INFLAMMATORY JOINT DISEASE**
PANTOTHENSÄURE-ENTHALTENDE ARZNEIMITTEL ZUR BEHANDLUNG VON ENTZÜNDLICHEN GELENKSERKRANKUNGEN
MEDICAMENTS A BASE D'ACIDE PANTOTHENIQUE POUR LE TRAITEMENT DES MALADIES INFLAMMATOIRES DES ARTICULATIONS

(30) Priority: 16.09.1999 GB 9921985
(43) Date of publication of application: 22.01.2003
(73) Proprietor: BRITANNIA PHARMACEUTICALS LIMITED, Redhill, Surrey RH1 6YS (GB)
(72) Inventor: SHERWOOD, Paul, London W1N 2PA (GB); DAVIES, David, Keith, West Wickham, Kent BR4 9DJ (GB)
(74) Representative: Blake, John Henry Francis
(86) International application number: PCT/GB00/03490
(87) International publication number: WO 01/019359

(56) References cited:
- CH-A- 682 803
- GB-A- 1 145 623
- GB-A- 2 286 528
- US-A- 4 568 547
- DATABASE CHEMABS [Online] CHEMICAL ABSTRACTS SERVICE, COLUMBUS, OHIO, US; HORIKIRI, KAZUYA ET AL: "Local irritation test on the intraarticular administration of dibucaine hydrochloride injection (Neo Vitacain) in rabbits" retrieved from STN Database accession no. 117:226235 XP002161981 & OYO YAKURI (1992), 44(3), 303-7 ,
- DATABASE BIOSIS [Online] BIOSCIENCES INFORMATION SERVICE, PHILADELPHIA, PA, US; 1981 MOISEENOK A G ET AL: "ANTI INFLAMMATORY AND COENZYMIC ACTIVITY OF PANTOTHENIC-ACID DERIVATIVES IN ADJUVANT ARTHRITIS" Database accession no. PREV198274077858 XP002161982 & KHIMIKO-FARMATSEVTICHESKII ZHURNAL, vol. 15, no. 6, 1981, pages 76-81, ISSN: 0023-1134
- DATABASE CHEMABS [Online] CHEMICAL ABSTRACTS SERVICE, COLUMBUS, OHIO, US; YURGILEVICH, V. P. ET AL: "Some prerequisites to the use of pantothenic acid preparations in the complex therapy of rheumatism" retrieved from STN Database accession no. 91:32794 XP002161983 & KHIM., BIOKHIM. FUNKTS. PRIMEN. PANTOTENOVOI KISLOTY, MATER. GRODN. SIMP., 4TH (1977), 167-9. EDITOR(S): MOISEENOK, A. G. PUBLISHER: IZD. NAUKA TEKHNIKA, MINSK, USSR. ,
- BEERS M. H. ET AL. : "The Merck Manual" April 1999 (1999-04) , MERK LABORATORIES , N.J. (USA) XP002161979 page 445 -page 447 page 446, column 2, last paragraph
- FERNANDEZ-PALAZZI F ET AL: "INTRAARTICULAR DEXAMETHASONE IN ADVANCED CHRONIC SYNOVITIS IN HEMOPHILIA" CLINICAL ORTHOPAEDICS,LIPPINCOTT, PHILADELPHIA, PA,US, no. 343, 1997, pages 25-29, XP000865956 ISSN: 0095-8654
- BOLLOW M ET AL: "CT-GUIDED INTRAARTICULAR CORTICOSTEROID INJECTION INTO THE SACROILIAC JOINTS IN PATIENTS WITH SPONDYLOARTHROPATHY: INDICATION AND FOLLOW-UP WITH CONTRAST-ENHANCED MRI" JOURNAL OF COMPUTER ASSISTED TOMOGRAPHY,US,NEW YORK, NY, vol. 20, no. 4, 1996, pages 512-521, XP000881197
- MARY EAGLESON: "Concise Encyclopedia Chemistry" 1994 , WALTER DE GRUYTER BERLIN , NEW YORK XP002161980 page 775-776

## Description

This invention relates to medicaments and their use in the alleviation of inflammation and pain in joints.

Pain or loss of movement in joints is a common occurrence, particularly among the elderly or those who have suffered damage to cartilage, bone surfaces or ligaments.

Standard methods of treatment include the administration of corticosteroids by injection into the site of the inflammation. However, relief tends to be temporary and long term use carries a number of contraindications.

Pantothenic acid (sometimes known as vitamin B5) and its salts have been used as a dietary supplement and for treatment of bronchial asthma, hay-fever, sinusitis and neurodermatitis.

CH 682803 discloses the intramuscular administration of pantothenic acid for the treatment of polyarthritis.

GB 11 45623 discloses the concomittant use of α-pantothenic acid and cysteine for the treatment of osteoarthritis.

It has now been found that pantothenic acid is of value in reducing inflammation when injected into the region of an affected joint.

According to the present invention there is provided use of pantothenic acid or a derivative thereof in the preparation of a medicament for administration by injection into the region of a joint for alleviation of inflammation or pain.

The pantothenic acid may be used in the form of a salt, e.g. the calcium salt. Pantothenic acid is a naturally occurring substance in plant and animal tissue. A particularly rich source is royal jelly obtainable from honey bee colonies. It may be used in its naturally occurring state or as a chemically pure material. Synthetic methods of preparations include those described in US Patent Nos. 2,780,645; 2,845,456 and 2,934,428. The free acid and its salts are optionally active and the dextro-rotatory isomer is preferred.

Conveniently, a medicament is prepared by dissolving the active ingredient, i.e. pantothenic acid or a derivative in a suitable solvent, e.g. water, and injecting the solution into the affected joint. The initial treatment may cause some pain and it may, therefore, be desirable to co-administer a local anaesthetic, e.g. lignocaine, at the same time or shortly before the injection of the pantothenic acid.

Other physiologically active materials may be co-administered, e.g. cysteine or glucosamine.

It may also be desirable to co-administer within the same treatment regime, a surface active phospholipid (SAPL) such as dipalmitoyl-phosphatidyl choline (DPPC) or phosphatidyl glycerol (PG). Preferably, a mixture of DPPC and PG is employed. A preferred protein-free SAPL composition comprising a blend of DPPC and PG is available from Britannia Pharmaceuticals Ltd of Brighton Road, Redhill under the trade mark 'Alec'. SAPL's such as 'Alec' are believed to act as a lubricant in joints, taking over to some extent the function of synovial fluid.

The medicaments of this invention may be used in the treatment of several conditions associated with inflammation or reduced movement of joints. Examples include tennis elbow, housemaid's knee, frozen shoulder, inflamed knee joints and hips and back pain associated with inflammation or restricted movement in the spinal vertebrae.

A typical dose is about 2 mls of an aqueous solution of about 500 mg of the acid salt. The medicament is injected directly into the site of the inflamed joint. A programme of injections is generally desirable in which dosages similar to that indicated above are given at intervals of a few days to about one week. Reduced inflammation and increased freedom of movement is usually noticeable after about a week from the initial injection.

## Claims

1. Use of pantothenic acid or a derivative thereof in the preparation of a medicament for administration by injection into the region of an inflamed or painful joint for alleviation of said inflammation or pain.

2. Use according to claim 1, wherein the derivative is a salt of pantothenic acid.

3. Use according to claim 2, wherein the salt is the calcium salt.

4. Use according to any one of the preceding claims, wherein the medicament comprises an aqueous solution of pantothenic acid or a derivative thereof.

5. Use according to any one of the preceding claims, wherein the medicament is to be co-administered with a local anaesthetic.

6. Use according to any one of the preceding claims, wherein the medicament is to be co-administered with cysteine or glucosamine.

7. Use according to any one of the preceding claims, wherein the medicament is to be co-admmistered with a surface-active phospholipids (SAPL).

8. Use according to claim 7, wherein the SAPL comprises dipalmitoylphosphatidyl choline.

9. Use according to claim 7 or 8, wherein the SAPL comprises phosphatidyl glycerol.

10. Use according to any one of claims 1 to 9 in which the joint is affected by a disorder selected from tennis elbow, housemaid's knee, frozen shoulder, inflamed knee joints and hips and back pain associated with inflammation or restricted movement in the spinal vertebrae.

## Patentansprüche

1. Verwendung von Pantothensäure oder eines daraus abgeleiteten Derivats für die Herstellung eines Medikaments, das zur Linderung einer Entzündung oder von Schmerzen mittels Injektion im Bereich eines entzündeten oder schmerzenden Gelenks verabreicht wird.

2. Verwendung nach Anspruch 1, wobei das Derivat ein Salz der Pantothensäure ist.

3. Verwendung nach Anspruch 2, wobei das Salz Calciumsalz ist.

4. Verwendung nach einem der vorangehenden Ansprüche, wobei das Medikament eine wässrige Lösung der Pantothensäure oder eines Derivats derselben umfasst.

5. Verwendung nach einem der vorangehenden Ansprüche, wobei das Medikament gemeinsam mit einer Lokalanästhesie zu verabreichen ist.

6. Verwendung nach einem der vorangehenden Ansprüche, wobei das Medikament gemeinsam mit Zystein oder Glucosamin zu verabreichen ist.

7. Verwendung nach einem der vorangehenden Ansprüche, wobei das Medikament gemeinsam mit einem oberflächenaktiven Phospholipid (SAPL) zu verabreichen ist.

8. Verwendung nach Anspruch 7, wobei das SAPL Dipalmitoylphosphatidylcholin enthält.

9. Verwendung nach Anspruch 7 oder 8, wobei das SAPL Phosphatidylglycerin enthält.

10. Verwendung nach einem der Ansprüche 1 bis 9, bei der das Gelenk von einer Beeinträchtigung betroffen ist, die ausgewählt ist aus Tennisellenbogen, Bursitis praepatellaris, fibröse Schultersteife, Kniegelenks- und Hüftgelenksentzündung sowie Rückenschmerzen in Verbindung mit einer Entzündung oder eingeschränkter Bewegung in den Rückenwirbeln.

## Revendications

1. Utilisation d'acide pantothénique ou d'un dérivé de celui-ci lors de la préparation d'un médicament pour l'administration par injection dans la zone d'une articulation enflammée ou douloureuse pour atténuer ladite inflammation ou douleur.

2. Utilisation selon la revendication 1, dans laquelle le dérivé est un sel de l'acide pantothénique.

3. Utilisation selon la revendication 2, dans laquelle le sel est le sel de calcium.

4. Utilisation selon l'une quelconque des revendications précédentes, dans laquelle le médicament comprend une solution aqueuse d'acide pantothénique ou d'un dérivé de celui-ci.

5. Utilisation selon l'une quelconque des revendications précédentes, dans laquelle le médicament doit être co-administré avec un anesthésique local.

6. Utilisation selon l'une quelconque des revendications précédentes, dans laquelle le médicament doit être co-administré avec de la cystéine ou de la glucosamine.

7. Utilisation selon l'une quelconque des revendications précédentes, dans laquelle le médicament doit être co-administré avec un phospholipide tensioactif (SAPL).

8. Utilisation selon la revendication 7, dans laquelle le SAPL comprend de la dipalmitoylphosphatidyl choline.

9. Utilisation selon la revendication 7 ou 8, dans laquelle le SAPL comprend du phosphatidyl glycérol.

10. Utilisation selon l'une quelconque des revendications 1 à 9, dans laquelle l'articulation est affectée par un trouble choisi parmi l'épicondylite des joueurs de tennis, l'hygroma prérotulien, l'épaule gelée, les articulations du genou et les hanches enflammées, et une douleur dorsale associée avec une inflammation ou un mouvement des vertèbres restreint.
